Europäisches Patentamt

European Patent Office

Office européen des brevets

(11) Veröffentlichungsnummer: **0 195 331 B1**

(12) **EUROPÄISCHE PATENTSCHRIFT**

(45) Veröffentlichungstag der Patentschrift: **06.05.92**

(51) Int. Cl.⁵: **C07K 15/00**, C12P 21/00,
C12N 5/00, C12N 15/00,
G01N 33/577, G01N 33/68,
C07K 3/18, A61K 49/00,
//(C12P21/00,C12R1:91)

(21) Anmeldenummer: **86103100.3**

(22) Anmeldetag: **08.03.86**

(54) Monoklonale Antikörper gegen atriale, natriurethische Peptide.

(30) Priorität: **20.03.85 DE 3509958**

(43) Veröffentlichungstag der Anmeldung:
**24.09.86 Patentblatt 86/39**

(45) Bekanntmachung des Hinweises auf die
Patenterteilung:
**06.05.92 Patentblatt 92/19**

(84) Benannte Vertragsstaaten:
**AT BE CH DE FR GB IT LI NL SE**

(56) Entgegenhaltungen:
**WO-A-85/04870**

**Biological Abstracts, Band 79, 1985 Zusammenfassung Nr 102221, Philadelphia, Pa US;
J Tang et al.: "Characterisation and localization of striopeptin in rat atrium"**

**Biological Abstracts, Band 78, 1984, Zusammenfassung Nr. 26435, Philadelphia, Pa, US;
K Kangawa et al.: "Purification and complete**
**amino acid sequence of beta-rat atrial natriuretic polypeptide of 5000 daltons"**

(73) Patentinhaber: **BAYER AG**

**W-5090 Leverkusen 1 Bayerwerk(DE)**

(72) Erfinder: **Hirth, Claudia, Dr.**
**Stockmannsmühle 127**
**W-5600 Wuppertal 1(DE)**
Erfinder: **Morich, Frank-Joachim, Dr.**
**Untere Bergerheide 15**
**W-5600 Wuppertal 1(DE)**
Erfinder: **Neuser, Dieter, Dr.**
**Am Braken 49**
**W-5603 Wülfrath(DE)**
Erfinder: **Stasch, Johannes-Peter, Dr.**
**Schneewittchenweg 37**
**W-5600 Wuppertal 1(DE)**

Rank Xerox (UK) Business Services
(-/2.18/2.0)

**Beschreibung**

Die Erfindung betrifft monoklonale Antikörper gegen atriale, natriuretische Peptide von Säugetieren, die keine Kreuzreaktivität mit anderen endogenen Mediatoren mit Gefäßwirkung bzw. mit Wirkung auf den Salz-Wasser-Haushalt haben, Verfahren zu deren Herstellung und deren Verwendung als Diagnostikum und für Forschungszwecke sowohl in vivo als auch in vitro.

Die Fusion von Maus-Myelom-Zellen mit Milzzellen von immunisierten Mäusen (Köhler und Milstein, Nature 256, 495-497 (1975)) war der erste Hinweis auf die Möglichkeit, kontinuierliche Zellinien zu erhalten, die einheitliche (sog. "monoklonale") Antikörper produzieren. Seitdem sind zahlreiche Versuche unternommen worden, verschiedene Hybridzellen (sog. "Hybridome") herzustellen und die Antikörper, die von ihnen gebildet werden, für unterschiedliche wissenschaftliche Untersuchungen einzusetzen (z.B.: Current Topics in Microbiology and Immunology, Vol. 81 - "Lymphocyte Hybridomas", F.Melchers et al., Springer Verlag, 1978 sowie Referenzen darin; C.Barnstable et al., Cell 14, 9-20 (1978); P.Parham, W.F.Bodmer, Nature 276, 397-399 Handbook of Experimental Immunology, 3. Auflage, Band 2, D.M.Wier, Herausgeber, Blackwell 1978, Kapitel 25; Chem. Eng. News, 15-17 (1979) 9). Diese Arbeiten schildern die prinzipielle Technik zur Produktion monoklonaler Antikörper durch Hybridome.

Monoklonale Antikörper gegen Histokompatibilitätsantigene, gegen Haptene, Proteine und Enzyme sind erzeugt worden. Es ist jedoch noch kein monoklonaler Antikörper bekannt, der hoch selektiv mit atrialen, natriuretischen Peptiden von Säugetieren wie zum Beispiel die vom Menschen oder der Ratte reagiert.

Bei den atrialen, natriuretischen Peptiden handelt es sich um eine Gruppe mit unterschiedlicher Kettenlänge, deren minimal wirksame Sequenz aus 23 Aminosäuren besteht. Peptide, die zusätzlich N-terminal und C-terminal weitere Aminosäuren enthalten, sind ebenfalls wirksam. Die Peptide entstehen durch enzymatische Einwirkungen im Herzvorhof aus einem Vorläufermolekül von ungefähr 152 Aminosäuren. Das für ANP kodierende Gen ist geklont, die DNA sequenziert.

Die ANP induzieren eine kurzdauerne, starke Natriurese, wobei bis heute ein exakter tubulärer Angriffspunkt der Substanzen nicht nachgewiesen werden konnte. Zusätzlich sind ANP potente Vasodilatatoren, die u.a. die Gefäßwirkung von Noradrenalin, Angiotensin II, Histamin und Serotonin antagonisieren können. Die wirkung von ANP wird nicht durch Indomethacin aufgehoben, was eine Wirkung über die endogene Prostaglandinsynthese unwahrscheinlich macht. Blutdrucksenkende Wirkungen von ANP sind in two-clip hypertonen Ratten und SH-Ratten beschrieben.

An isolierten Zellen konnte eine inhibierende Wirkung auf die Aldosteron- und Vasopressin-Sekretion nachgewiesen werden. Unter Benutzung von konventionellen Antikörpern lassen sich radioimmunologisch Plasmaspiegel von ANP messen, die vom Volumenzustand des jeweiligen Versuchstieres abhängig sind. Mit einem $^{125}$Jod-markierten ANP-Derivat ließ sich eine spezifische Bindung an isolierte Zona glomerulosa-Zellen nachweisen. Erste Experimente deuten auf eine Freisetzung von Kallikrein in der Niere durch ANP. Unter bestimmten Bedingungen kann die Nierenwirkung der Peptide durch Vorbehandlung mit Haloperidol aufgehoben werden (Sagnella und MacGregor, Nature 399, 666-668, 1984). Das Wirkungsspektrum von ANP macht eine kausale oder symptomatische Beteiligung dieser Peptide bei Kreislauferkrankungen (Hypertonie, Hypotonie, Arteriosklerose), bei Herzerkrankungen, (akute und chronische Herzinsuffizienz, Herzinfarkt, Herzrhythmusstörungen, coronare Herzerkrankung) und bei Nierenerkrankungen (akutes und chronisches Nierenversagen im Verlauf unterschiedlicher Grunderkrankungen, Urämie) sehr wahrscheinlich. Bei allen genannten Krankheitsbildern wird deshalb die quantitative Bestimmung von ANP in verschiedenen biologischen Körperflüssigkeiten (z.B. Blut, Plasma, Serum, Urin, Lymphe oder in der cerebrospinalen Flüssigkeit) eine große Bedeutung erlangen.

Die mit herkömmlichen Antiseren gemessenen Plasmaspiegel von ANP sind z.T. sehr hoch, was durch die Kreuzreaktivität bzw. mangelnde Spezifizität dieser Antikörper erklärt werden kann. Die Gefahr der Bestimmung falsch hoher Plasmaspiegel an atrialen, natriuretischen Peptiden läßt sich durch den Einsatz hochspezifischer (monoklonaler) Antikörper ausschließen. Damit wären solche Antikörper ideal für die Diagnostik aller Erkrankungen mit verändertem ANP-Spiegel geeignet.

Die vorliegende Erfindung betrifft die Herstellung von Hybridom-Zellinien, die hochspezifische, monoklonale Antikörper gegen atriale, natriuretische Peptide synthetisieren und sezernieren, wobei diese Antikörper keine Kreuzreaktivität mit anderen endogenen Mediatoren mit Gefäßwirkung bzw. Wirkung auf den Salz-Wasser-Haushalthaben. Die Erfindung betrifft weitertun Hybridomzellen, die monoklonale Antikörper gegen atriale, natriuretische Peptide von Säugetieren insbesondere gegen die vom Menschen oder der Ratte synthetisieren und sezernieren. Diese Hybridome werden nach der eingangs genannten Methode von Köhler und Milstein hergestellt. Nach der Immunisierung von Mäusen mit an einen immunogenen Träger wie z.B. Protein gebundene atriale, natriuretische Peptide werden die Milzzellen dieser Mäuse mit Zellen einer Maus-Myelom- Zellinie fusioniert. Die daraus resultierenden Hybridome werden systematisch auf Antikörper untersucht, die selektiv mit den atrialen, natriuretischen Peptiden reagieren. Auf diese Weise wurden Hybridome isoliert, die Antikörper gegen atriale, natriuretische Peptide produzieren. Die Erfindung betrifft weiterhin diese Antikörper.

Hybridome, die diesen Antikörper produzieren wurden unter der Hinterlegungsnummern 85031401 (Hybridomzellinie die den mAk 11A-A11 produziert) und 85031402 (Hybridomzellinie die den mAk 23 M-D9 produziert) bei der National Collection of Animal Cell Cultures, PHLS Centre for Applied Mikrobiology and Research, Porton Down, Salisbury SP4 OJG, UK am 14.03.1985 hinterlegt.

Die erfindungsgemäßen Antikörper können z.B. zur Bestimmung der Spiegel an atrialen, natriuretischen Peptiden in biologischen Flüssigkeiten wie z.B. Blut, Plasma, Serum, Urin, Lymphe oder in der cerebrospinalen Flüssigkeit verwendet werden. Die erfindungsgemäßen Antikörper sind für die Herstellung von Immunoassays besonders gut geeignet. Sie können aber auch zur Isolierung von atrialen, natriuretischen Peptiden mittels Immunadsorptionschromatographie eingesetzt werden.

Für die Erforschung der Bedeutung von ANP unter unterschiedlichen physiologischen und pathophysiologischen Bedingungen sind tierexperimentelle Untersuchungen unbedingt notwendig. Voraussetzung für diese Arbeiten und für alle Arbeiten zur Pharmakologie von ANP sind spezifische und sensible Tests zur quantitativen Bestimmung in Körperflüssigkeiten.

Die erfindungsgemäßenb Antikörper können z.B. zur Bestimmung der Spiegel an atrialen, natriuretischen Peptiden im Serum von Versuchstieren für diagnostische Zwecke verwendet wurden. Für physiologische oder pharmakologische Experimente sind die erfindungsgemäßen Antikörper auch sehr gut geeignet.

Die Herstellung der Hybridome umfaßt im allgemeinen die folgenden Schritte:

A) Immunisierung von Säugetieren mit an immunogene Träger, insbesondere an immunogenes Trägerprotein (z.B. Keyhole Limpet Hemocyanin) gebundenem atrialen, natriuretischen Peptid wie zum Beispiel vom Menschen oder der Ratte (KLH-ANP). Weibliche Balb/c-Mäuse erwiesen sich hierzu als brauchbar, jedoch können auch andere Mäusestämme verwendet werden. Das Immunisierungsschema und die Konzentrationen KLH-ANP sollten so gewählt werden, daß eine hinreichende Anzahl antigen-stimulierten Lymphozyten gebildet wird. Drei Immunisierungen im Abstand von 14 Tagen mit 100 μg KLH-ANP/Maus durch Injektion in Phosphat gepufferter physiologischer Kochsalzlösung erwiesen sich als effektiv.

B) Gewinnung der Milzen der immunisierten Säugetiere (z.B. Mäuse) und Herstellung einer Milzzellsuspension in einem geeigneten Medium. Ungefähr 1 ml Medium/Milz ist ausreichend. Die experimentellen Techniken dazu sind bekannt.

C) Fusion der suspendierten Milzzellen mit Myelomzellen einer geeigneten Zellinie (z.B. Mausmyelom PX63Ag8), indem man einen geeigneten Fusionspromotor (z.B. Polyethylenglykol) verwendet. Bevorzugte Fusionspromotoren sind Polyethylenglykole von mittlerem Molekulargewicht zwischen 1000 und 4000 (z.B. kommerziell erhältliches PEG 1000 etc.). Jedoch können auch andere bekannte Fusionspromotoren benutzt werden. Bevorzugt wird ein Verhältnis von etwa 10 Milzzellen/Myelomzellen. Ein Gesamtvolumen von etwa 0,5-1,0 ml Fusionsmedium ist ausreichend für $10^8$ Milzzellen. Es sind viele Myelomzellen aus Mäusen bekannt und erhältlich, z.B. von wissenschaftlichen Instituten oder Zellhinterlegungsinstitutionen. Die verwendete Zellinie soll vorzugsweise einen genetischen Defekt aufweisen, so daß nicht-fusionierte Myelomzellen in einem Selektionsmedium absterben, während Hybride überleben. Am häufigsten werden 8-Azaguanin-resistente Zellinien, welchen das Enzym Hypoxanthin- Guanin-Phosphoribosyl-Transferase fehlt, und die deshalb in einem HAT-Medium (Hypoxanthin, Aminopterin, Thymidin) nicht wachstumsfähig sind, benutzt (Science 145:709, 1964).

Vorzugsweise soll die verwendete Myelomzellinie auch vom "non-secreting"-Typ sein, damit sie nicht selbst Antikörper oder H- bzw. L-Ketten von Immunglobulinen bildet. In gewissen Fällen können jedoch sezernierende Myelomzellen von Vorteil sein.

D) Das Fusionsgemisch (Milz- und Myelomzellen) wird in Einzelgefäßen in einem Selektivmedium verdünnt und kultiviert, so daß die nicht-fusionierten Zellen sich nicht vermehren und in 1-2 Wochen absterben. Die einzelnen fusionierten Zellen werden isoliert, indem das Volumen des Verdünnungsmittels so eingestellt wird, daß eine bestimmte Zahl von Zellen (etwa 1-4) in das einzelne Gefäß (z.B. jede Vertiefung einer Mikrotiterplatte) kommt.

E) Überprüfung auf Anwesenheit von Antikörpern gegen atriale, natriuretische Peptide in jedem Gefäß.

F) Selektion (z.B. durch limitierende Verdünnung) und Klonierung der Hybridome, die den gewünschten Antikörper produzieren.

Ist einmal das gewünschte Hybridom selektioniert und kloniert worden, kann man den Antikörper auf zwei verschiedenen Wegen produzieren. Monoklonale Antikörper von sehr hoher Reinheit erhält man, wenn man die Hybridome in einem geeigneten Medium über eine gewisse Zeit kultiviert und den Antikörper aus dem Überstand gewinnt. Ein geeignetes Medium und die optimale Kulturdauer sind einfach zu bestimmen. Diese in vitro-Technik liefert monoklonale Antikörper, die nur mit geringen Mengen von Proteinen aus dem heterologen Serum (z.B. fötalem Kälberserum) verunreinigt sind.

Um eine wesentlich höhere Konzentration an monoklonalen Antikörpern mit nur wenig geringerer Reinheit herzustellen, kann man das ausgewählte Hybridom einer Maus - vorzugsweise einer syngenen oder semisyngenen - intraperitoneal injizieren. Dies führt in der Maus nach einer Inkubationszeit zur Bildung eines Tumors, der hohe Antikörperkonzentrationen (5-20 mg/ml) im Blut und im peritonealen Exsudat (Ascites) des Wirtstieres freisetzt. Wenn auch diese Mäuse normale Antikörper im Blut und im Ascites haben so ist deren Konzentration im Vergleich zu den mAK sehr gering. Der so gewonnene monoklonale Antikörper hat einen hohen Titer (er ist aktiv in Verdünnungen von $10^{-3}$ oder darunter), und das Verhältnis zwischem spezifischem und nicht-spezifischem Immunglobulin beträgt etwa 20:1.

### Beispiel 1

Herstellung der monoklonalen Antikörper gegen atriale, natriuretische Peptide vom Menschen und der Ratte

### Herstellung des zur Immunisierung verwendeten Antigens

Als Antigen wurde ein Konjugat von humanem $\alpha$-ANP bzw. Atriopeptin II der Ratte (Firma Bachem) und Keyhole Limpet Hemocyanin (KLH, Pacific Biomarine Supply Company) verwendet. Zur Herstellung wurden KLH und humanes $\alpha$-ANP bzw. Atriopeptin II im molaren Verhältnis von 1:1 gemischt und bei einer Konzentration von 2 mg/ml in Phosphat gepufferter physiolog. Kochsalzlösung mit Glutaraldehyd versetzt. Die Endkonzentration von Glutaraldehyd im Reaktionsansatz betrug 0,25 %. Nach einer Stunde Inkubation bei Raumtemperatur wurden die Proteinkonjugate bei 4°C mehrmals gegen Phosphat gepufferte physiolog. Kochsalzlösung dialysiert.

### Immunisierung von Balb/c-Mäusen

Weibliche Balb/c-Mäuse wurden intraperitoneal mit 100 $\mu$g KLH-ANP-Konjugat in 0,2 ml kompletten Freund'schen Adjuvans immunisiert. 14 Tage später erhielten die Tiere erneut 100 $\mu$g des Antigens intraperitoneal in inkomplettem Freund'schen Adjuvans. Zwei weitere Immunisierungen erfolgen intravenös in 14-tägigen Abständen mit jeweils 50 $\mu$g Antigen in 100 $\mu$g Phosphat gepufferter Kochsalzlösung/Tier. Drei Tage nach der letzten Antigengabe wurden die Milzen der Tiere entnommen.

### Präparation einer Milzzellsuspension

Aus den entnommenen Milzen wurden Einzelzellsuspensionen hergestellt, indem die Organe durch ein Sieb aus rostfreiem Stahl gepreßt wurden. Die Zellen wurden in Dulbecco's Minimal Essential Medium (DMEM), das mit 4,5 g/l Glukose, 100 Einheiten/ml Penicillin und 100 $\mu$g/ml Streptomycin supplementiert war, überführt. Die Zellen wurden 3mal mit DMEM gewaschen und dann in gewünschter Konzentration im selben Medium resuspendiert. Im allgemeinen wurden etwa $10^8$ Zellen/Milz gewonnen.

### Präparation der Myelomzellen

Die hier verwendete Myelomzellinie X63Ag8.653 ist ein Subklon der Mäusemyelomzellinie P3-X63-Ag8, der keine schweren oder leichten Ketten von Immunglobulinen exprimiert (J. Immunol. 123:1543-1550 (1980)). Die Zellen sind gegen 20 $\mu$g/ml 8-Azaguanin sensitiv und können in Medium, das Hypoxanthin, Aminopterin und Thymidin (HAT) enthält, nicht mehr wachsen. Sie wurden in DMEM. das mit 4,5 g/l Glukose, 20 mM Glutamin, 1000 Einheiten/ml Penicillin, 100 $\mu$g Streptomycin und 10 % fötalem Kälberserum supplementiert war (komplettes Medium), kultiviert. Die Myelomzellen waren zum Zeitpunkt der Fusionierung in der logarithmischen Phase der Zellvermehrung.

### Zellfusion

Die Milzzellsuspensionen wurden zu den Myelomzellen in DMEM ohne Serum mit einem Verhältnis von 10:1 zugegeben und 10 min bei 200 g in Bechern mit rundem Boden zentrifugiert. Nach Absetzen der Zellen wurde der Überstand vorsichtig dekantiert. Die Zellen wurden mit 2 ml einer Lösung von Polyethylenglykol vom Molekulargewicht 2000 (verdünnt auf 30 % w/w mit DMEM, pH 7,6) 1 min bei 37 ° C inkubiert. Daraufhin wurden 20 ml DMEM zugegeben, worauf die Zellen über einige Minuten vorsichtig resuspendiert wurden. Sie wurden nochmals 5 min lang bei 200 g zentrifugiert und das Zellpellet in HAT-Medium in einer Konzentration von $10^6$ Zellen/ml resuspendiert, worauf sie in 1 ml-Anteilen auf Costarplatten verteilt wurden.

## Selektionierung und Anzucht der Hybridome

Nach der Zellfusion wurden die Zellen in HAT-Medium (Hypoxanthin, Aminopterin, Thymidin) bei 37 ° C mit 5 % $CO_2$ in einer feuchten Atmosphäre kultiviert. Nach einigen Wochen wurden Überstände von Hybridomzellkulturen auf das Vorhandensein von anti-ANP-Aktivität mit Hilfe des entsprechenden Enzymimmunoassays untersucht (s. unten).

Die jenigen Hybridomzellinien, die positive Resultate im anti-ANP-Test zeigten, wurden zum Klonieren ausgewählt.

Dabei wurden die Hybridome einer "Limiting-Dilution"-Technik unterworfen, bei der in 96 Mikrotitervertiefungen im Mittel 0,5 Zellen/Vertiefung ausgesät wurden, wobei $10^5$ Mäusethymocyten/Vertiefung als "Feeder-Zellen" zugegeben wurden. Die nach diesem Klonierungsverfahren noch anti-ANP-Antikörper-produzierenden Zellen wurden vermehrt, eingefroren und in flüssigem Stickstoff in komplettem Medium, das 25 % fötales Kälberserum sowie 7,5 % Dimethylsulfoxid enthielt, aufbewahrt.

## Herstellung großer Mengen monoklonaler Antikörper

Klonierte Hybridome wurden Mäusen, die durch intraperitoneale Injektion mit 0,5 ml Pristan vorbehandelt waren, intraperitoneal injiziert, um antikörperenthaltende Ascitesflüssigkeit nach ca. 10 bis 15 Tagen gewinnen zu können. Die Antikörperaktivität in der Ascitesflüssigkeit wurde in einem radioaktiven Bindungsinhibitionstest (siehe Beispiel 3) bestimmt.

## Bestimmung der anti-ANP-Aktivität in Kulturüberständen

100 $\mu$l Kulturüberstand wurden auf Mikrotiterplatten gegeben, an die ein Konjugat aus bovinem Serumalbumin und humanem $\alpha$-ANP bzw. Atriopeptin II der Ratte (5 $\mu$g/ml) 3 Stunden bei Raumtemperatur absorbiert worden war, und die daraufhin mit Wasser 5mal gewaschen worden waren. Kaninchen-anti-Maus-Immunglobulin, das mit Peroxydase gekuppelt war, wurde daraufhin zugefügt; danach wurden die Platten 2 Stunden bei Zimmertemperatur inkubiert. Nach 5maligem waschen mit destilliertem Wasser wurde ein für das Enzym geeigneter Substratpuffer hinzugegeben, und die Konzentration des entstehenden gefärbten Produktes mit einem geeigneten Photometer für Mikrotiterplatten bestimmt.

## Beispiel 2

Charakterisierung des monoklonalen Antikörpers gegen atriale, natriuretische Peptide

## Typisierung des monoklonalen Antikörpers

Die Klassen und Unterklassen des hier beschriebenen monoklonalen Antikörpers wurden analysiert. Der Antikörper wurde aus dem Kulturüberstand durch Fällung mit Ammoniumsulfat (40 %ige Sättigung) angereichert und partiell gereinigt. Unter Verwendung von klassenspezifischen anti-Maus-Immunglobulin-Antiseren wurde die Immunglobulinklasse im Ouchterlony Geldiffusionstest bestimmt. Der monoklonale Antikörper 23M-D9 gegen ANP vom Menschen gehört zur IgG$_1$-Klasse. Der monoklonale Antikörper 11A-A11 gegen Atriopeptin II gehört ebenfalls zur IgG$_1$-Klasse.

## Kreuzreaktion des Antikörpers mit verschiedenen anderen Peptiden

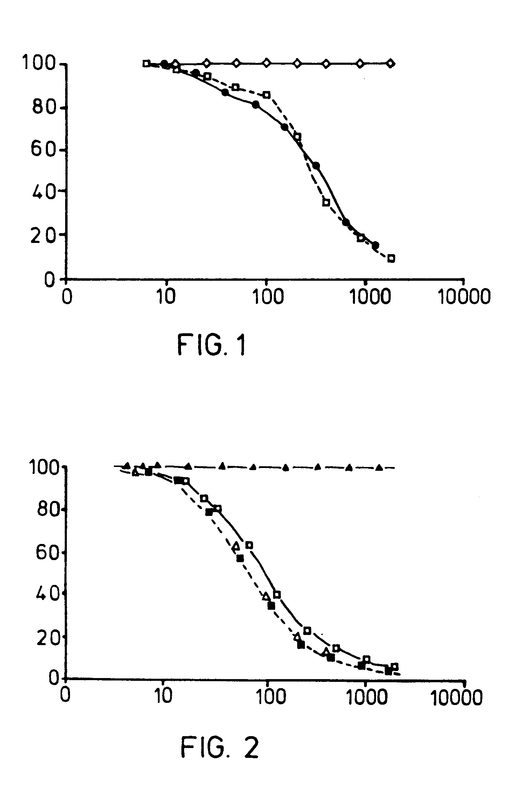

Die Kreuzreaktivität des monoklonalen Antikörpers gegen humanes ANP mit verschiedenen anderen Peptiden wurde mit Hilfe eines radioimmunologischen Bindungsinhibitionstests ermittelt. Der Test wird in einem speziellen Assaypuffer (0,02 M Natrium-Phosphat; 0,15 M NaCl; 0,01 % Thiomersal; 0,1 % Gelatine; 0,01 % BSA und 0,1 % Triton-X 100, pH 7,4) durchgeführt. Radioaktiv markiertes [125]Jod-$\alpha$-Human-ANP (Aminosäuren 1-28) vom Menschen bzw. [125]Jod-Atriopeptin II der Ratte (Amersham Buchler) werden zusammen mit Antikörpern in geeigneter Verdünnung für 16-48 Std. bei 4 ° C inkubiert. Das Gesamtvolumen beträgt 500 $\mu$l. Beim Bindungs-Inhibitionstest enthält der Testansatz bei gleichem Volumen zusätzlich unmarkiertes Antigen bzw. die verschiedenen Inhibitoren in unterschiedlichen Konzentrationen. Am Ende der Inkubation wird das nicht an den Antikörper gebundene, radioaktiv markierte [125]Jod-Antigen (Human-α-ANP mit den Aminosäuren 1-28 bzw. Atriopeptin 11 der Ratte) nach Zugabe einer Aktivkohlesuspension (0,02 M Natriumphosphat; 2 % Norit-A-Aktivkohle; 0,02 % Dextran 60; 0,1 % BSA und 10 mM $Na_2$-EDTA, pH 7,4) und anschließender Inkubation von 20 min auf Eis an die Kohle adsorbiert. Danach wird die Aktivkohle durch Zentrifugation für 10 min bei 3600 g sedimentiert und die Radioaktivität im Überstand bestimmt. Gemessen wurde die Konzentration von verschiedenen Peptiden, die erforderlich ist, um 50 % des [125]Jod-Antigen vom Antikörper zu verdrängen. Wie aus der Abbildungen 1 und 2 hervorgeht, reagieren die Antikörper hochspezifisch mit atrialen Peptiden vom Menschen und der Ratte. Kreuzreaktivitäten mit anderen an der Regulation des Salz-Wasser-Haushaltes beteiligten Mediatoren ließen sich nicht nachweisen. Genau so unwirksam waren verschiedene vasoaktive Substanzen (siehe Tabellen 1 und 2).

## Beispiel 3

Anwendung des monoklonalen Antikörpers gegen atriale, natriuretische Peptide

Nachweis von ANP-Immunreaktivität in biologischen Flüssigkeiten und Geweben mit dem monoklonalen Antikörper

Antigene in hoher Verdünnung lassen sich quantitativ mit Hilfe des Radioimmunoassays bestimmen. Erforderlich für diesen Test sind hochspezifische Antikörper und ein radioaktiv markiertes Antigen. Die Bindung des radioaktiv markierten Antigens an den Antikörper läßt sich dosisabhängig durch nicht radioaktives Antigen inhibieren. Werden definierte, unterschiedliche Konzentrationen nicht radioaktiven Antigens mit konstanten Konzentrationen an Antikörpern und radioaktiv markiertem Antigen inkubiert, so läßt sich auf diese Weise eine charakteristische Eichkurve erstellen. Die Abbildung 1 zeigt eine solche Eichkurve und zwar die Verdrängung des radioaktiv markierten Antigens ([125]Jod-Human-alpha-ANP, Aminosäuren 1-28) vom mAK 23M-D9 durch verschiedene Atriopeptine bzw. deren Derivate. Dabei wurde die Bindung des radioaktiven Antigens an den Antikörper in % gegen die Konzentration an nicht markierten Antigen bzw. dessen Fragmente in fmol/ml aufgetragen. Dabei bedeutet

Human-α-ANP (1-28)

Human-ANP-Fragment (7-28)

ANP-Fragment (18-28)

Der Test wurde mit Ascitesflüssigkeit in einer Endverdünnung von 1:1500000 wie im Text beschrieben durchgeführt. Der Test mit Kulturüberstand liefert gleiche Ergebnisse, nur ist die einsetzbare Verdünnung um den Faktor 500 bis 1000 geringer. Dieses Experiment zeigt deutlich, daß das ANP-Fragment (Aminosäuren 18-28) nicht in der Lage ist das radioaktiv markierte Antigen von Antikörper zu verdrängen. Der Antikörper besitzt also keine Kreuzreaktivität mit diesem Fragment. Unbekannte Mengen an Antigen in Lösung können mit Hilfe dieser Eichkurve bestimmt werden. Ein Radioimmunoassay basierend auf dem hier beschriebenen monoklonalen Antikörper und [125]Jod-human-α-ANP (Aminosäuren 1-28) als Tracer ermöglicht den Nachweis in biologischen Flüssigkeiten und Gewebeextrakten bis zu einer Konzentration von 2O pg/ml, was ungefähr einer Menge von 6 fmol/ml entspricht (siehe Abbildung 1).

Die Abb. 2 zeigt ein ebenso durchgeführtes Experiment mit dem mAK 11A-A11, die spezifisch mit Atriopeptin der Ratte reagiert. Dabei bedeutet

Atriopeptin I

Atriopeptin II

Atriopeptin III

ANP-Fragment (13-28)

Die Auftragung ist die gleiche wie in Abbildung 1.

Der Test wurde mit Ascitesflüssigkeit in einer Endverdünnung von 1:1250000 wie im Text beschrieben durchgeführt. Der Test mit Kulturüberstand liefert gleiche Ergebnisse, nur ist die einsetzbare Verdünnung um den Faktor 500 bis 1000 geringer. Dieses Experiment zeigt deutlich, daß das ANP-Fragment (Aminosäuren 13-28) nicht in der Lage ist das radioaktiv markierte Antigen von Antikörper zu verdrängen. Der Antikörper besitzt also keine Kreuzreaktivität mit diesem Fragment. Unbekannte Mengen an Antigen in Lösung können mit Hilfe dieser Eichkurve bestimmt werden. Ein Radioimmunoassay basierend auf dem hier beschriebenen monoklonalen Antikörper und [125]Jod- Atriopeptin III als Tracer ermöglicht den Nachweis in biologischen Flüssigkeiten und Gewebeextrakten bis zu einer Konzentration von 20 pg/ml, was ungefähr einer Menge von 6 fmol/ml entspricht (siehe Abbildung 2).

Mit den beschriebenen monoklonalen Antikörpern sind neben dem hier dargestellten radioimmunologischen verfahren auch andere Formen des Immunoassays (z.B. ELISA, Chemoluminiszenz) durchführbar.

Beispiel 4

Antagonisierung der in vivo-Effekte von Atriopeptin der Ratte

Verwendet wurden männliche Wistar-Ratten (Körpergewicht 240-270 g), die am Abend vor dem Versuchstag nüchtern gesetzt wurden, aber freien Zugang zum Trinkwasser hatten. Präparation und Versuch erfolgten in Inactin-Narkose (100 mg/kg i.p.). Nach Tracheotomie und Injektion von 1 mg/kg Atropin i.p. wurden zur Blutdruckmessung ein Plastik-Katheter in die Arteria femoralis und zur Injektion bzw. Infusion von Lösungen ein Plastik-Katheter in die Vena jugularis eingebunden sowie ein Blasenkatheter gelegt. Nach Abschluß der Präparation erhielten die Ratten eine Injektion von 5 ml/kg 0,9 %iger NaCl-Lösung und anschließend für die Dauer eines Versuchs eine Infusion derselben Lösung (1,2 ml/Stunde). Die Rektaltemperatur der Versuchstiere wurde durch Wärmestrahler auf 37 ± 1°C gehalten. Nach Ablauf einer 1-stündigen Equilibrierungszeit wurde der Harn in vorher gewogenen Gefäßen gesammelt, die jeweils nach 10 bzw. 20 min gewechselt wurden. Die Konzentrationen von $Na^+$ und $K^+$ im Harn wurden flammenphotometrisch bestimmt (Instrumentation Laboratory, Lexington, Mass./USA). Die Antikörperflüssigkeit enthielt in 1 ml 0,9 %iger NaCl-Lösung 100 $\mu$l sterilgefilterter Ascitesflüssigkeit und 0,1 % Rinderserumalbumin. Synthetisches Atriopeptin II (Firma Bachem) wurde ebenfalls in einer wäßrigen Lösung mit 0,9 % NaCl und 0,1 % Rinderserumalbumin injiziert. Das Injektionsvolumen betrug in allen Fällen 1 ml/kg Körpergewicht. Vorversuche hatten gezeigt, daß die Bolusinjektion tion von 1 ml/kg Körpergewicht 0,9 %iger NaCl-Lösung mit 0,1 % Rinderserumalbumin Harnvolumen und Natrium-Ausscheidung nur geringfügig beeinflussen. Nach Abschluß der Versuche wurde von jeder Ratte ein arterielle Blutprobe zur Kontrolle des Säure-Basen-Status entnommen.

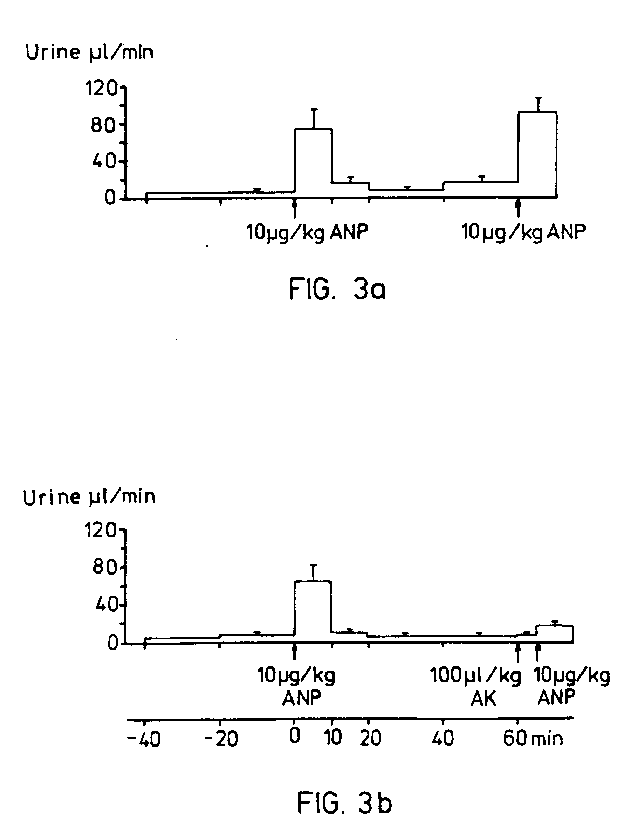

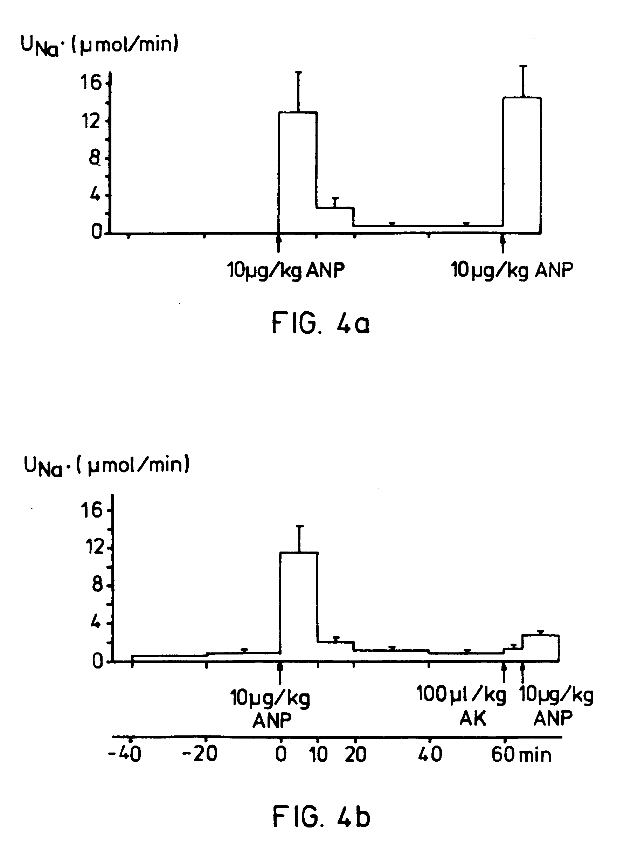

10 $\mu$g/kg Atriopeptin II führen in der ersten 10 min nach i.v. Bolusinjektion zu einer starken Zunahme von Harnvolumen und Natrium-Ausscheidung (s. Abb. 3 und 4). Diese Zunahme klingt in den darauffolgenden Sammelperioden rasch ab und läßt sich nach einer Stunde durch erneute Bolusinjektion von 10 $\mu$g/kg Körpergewicht ANP i.v. gut reproduzieren. Durch Bolusinjektion von 100 $\mu$l/kg Körpergewicht antikörperhaltiger Ascitesflüssigkeit (Antikörper 11A-A11) 5 min vor der zweiten ANP-Injektion läßt sich der Anstieg von Harnvolumen und Natrium-Ausscheidung fast vollständig unterdrücken.

Die Abbildungen 3 und 4 zeigen die Ergebnisse im einzelnen. Auf der Ordinate ist die Sammelzeit (Sammelperioden) in Minuten gegen die Ausscheidung von Urin in $\mu$l/min (Abb. 3a und 3b) bzw. die Natriumausscheidung in $\mu$mol/min (Abb. 4a und 4b) aufgetragen.

Abb. 3a:    Urinvolumen nach zweimaliger Bolusinjektion von 10 $\mu$g/kg Atriopeptin II (ANP), n = 4

Abb. 3b:    Antagonisierung des Effektes von 10 $\mu$g/kg Atriopeptin II durch mAK gegen ANP: 5 min vor der zweiten Injektion ANP wurde eine Bolusinjektion von 100 $\mu$l/kg antikörperhaltiger Ascitesflüssigkeit (AK) gegeben, n = 4

Abb. 4a:    Natriumausscheidung nach zweimaliger Bolusinjektion von 10 $\mu$g/kg Atriopeptin II (ANP), n = 4

Abb. 4b:    Antagonisierung des Effektes von 20 $\mu$g/kg Atriopeptin II durch mAK gegen ANP: 5 min vor der zweiten Injektion von ANP wurde ein Bolusinjektion von 100 $\mu$l/kg antikörperhaltiger Ascitesflüssigkeit (AK) gegeben, n = 4.

Die Antikörper antagonisieren also die natriuretische Wirkung von Atriopeptin II in vivo.

Tabelle 1

| Untersuchung der Kreuzreaktivität von mAk 23 M-D9 mit anderen endogenen Mediatoren mit Gefäßwirkung bzw. mit Wirkung auf den Salz-Wasser-Haushalt | | |
|---|---|---|
| Substanz | Max. getestete Kon. (pmol/ml) | Kreuzreaktivität |
| Angiotensin II (human) | 14 | - |
| Leu-Enkephalin | 26 | - |
| Substanz P | 10 | - |
| Aldosteron | 44 | - |
| ACTH (Schwein) | 4 | - |
| Bradykinin | 13 | - |
| $\gamma$-MSH | 10 | - |
| Insulin (Ratte) | 3 | - |
| Vasopressin | 15 | - |
| Renin (Schwein) | 0,5 | - |

Tabelle 2

| Untersuchung der Kreuzreaktivität von mAK 11A-A11 mit anderen endogenen Mediatoren mit Gefäßwirkung bzw. mit Wirkung auf den Salz-Wasser-Haushalt. | | |
|---|---|---|
| Substanz | Max. getestete Konz. (pmol/ml) | Kreuzreaktivität |
| Angiotensin II (human) | 14 | - |
| Leu-Enkephalin | 26 | - |
| Substanz P | 10 | - |
| Aldosteron | 44 | - |
| ACTH (Schwein) | 4 | - |
| Bradykinin | 13 | - |
| $\gamma$-MSH | 10 | - |
| Insulin (Ratte) | 3 | - |
| Vasopressin | 15 | - |
| Renin (Schwein) | 0,5 | - |

**Patentansprüche**

1. Monoklonale Antikörper gegen atriale, natiruretische Peptide von Säugetieren, dadurch gekennzeichnet, daß diese keine Kreuzreaktivität mit anderen endogenen Mediatoren mit Gefäßwirkung bzw. mit Wirkung auf den Salz-Wasser-Haushalt haben.

2. Monoklonale Antikörper gemäße Anspruch 1, wobei die endogenen Mediatoren Angiotensin II (human), Leu-Enkephalin, Substanz P, Aldosteron, ACTH (Schwein), Bradykinin, $\gamma$-MSH, Insulin (Ratte), Vaso-pressin und Renin (Schwein) sind.

3. Monoklonale Antikörper gemäß den Ansprüchen 1 und 2 produziert durch die Hybridomazelle 85031401.

4. Monoklonale Antikörper gemäß den Ansprüchen 1 und 2 produziert durch die Hybridomazelle 85031402.

5. Hybridomzellinien, dadurch gekennzeichnet, daß sie monoklonale Antikörper gemäß den Ansprüchen 1 bis 4 produzieren.

**6.** Verfahren zur Herstellung von monoklonalen Antikörpern gemäß den Ansprüchen 1 bis 4, dadurch gekennzeichnet, daß Hybridomzellinien gemäß Anspruch 5 verwendet werden.

**7.** Verfahren gemäß Anspruch 6, dadurch gekennzeichnet, daß man die Hybridomzellen in einem geeigneten Kulturmedium kultiviert und die Antikörper aus dem Überstand gewinnt.

**8.** Verfahren gemäß Anspruch 6, dadurch gekennzeichnet, daß man die Hybridomzellen syngenen oder semisyngenen Mäusen injiziert und die Antikörper aus dem Blut oder dem peritonealen Exsudat gewinnt.

**9.** Verfahren zur Herstellung von Hybridomzellen gemäß Anspruch 5, dadurch gekennzeichnet, daß Myelomzellen mit Zellen aus Säugetieren fusioniert werden, die Antikörper gegen atriale, natriuretische Peptide produzieren.

**10.** Verfahren gemäß Anspruch 9, dadurch gekennzeichnet, daß Milzzellen von gegen atriale natriuretische Peptide immunisierten Säugetieren mit Myelomzellen fusioniert werden.

**11.** Verfahren gemäß den Ansprüchen 9 und 10, dadurch gekennzeichnet, daß Milzzellen von gegen atriale natriuretische Peptide immunisierten Mäusen mit Myelomzellen fusioniert werden.

**12.** Verfahren gemäß den Ansprüchen 9 bis 11, dadurch gekennzeichnet, daß Mausmyelomzellen verwendet werden.

**13.** Verfahren gemäß den Ansprüchen 9 bis 12, dadurch gekennzeichnet, daß die Myelomzellen vom non-secreting-Typ sind.

**14.** Verfahren gemäß den Ansprüchen 9 bis 13, dadurch gekennzeichnet, daß die Immunisierung mit an einen immunogenen Träger gebundenen atrialen natriuretischen Peptiden erfolgt.

**15.** Verfahren gemäß den Ansprüchen 9 bis 14, dadurch gekennzeichnet, daß der immunogene Träger ein Protein ist.

**16.** Verwendung von monoklonalen Antikörpern gemäß den Ansprüchen 1 bis 4 zur Bestimmung von atrialen, natriuretischen Peptiden in biologischen Flüssigkeiten.

**17.** Verwendung von monoklonalen Antikörpern gemäß den Ansprüchen 1 bis 4 zur Bestimmung von atrialen, natriuretischen Peptiden in Blut, Plasma, Serum, Urin, Lymphe oder in der cerebro-spinalen Flüssigkeit.

**18.** Verwendung von monoklonalen Antikörpern gemäß den Ansprüchen 1 bis 4 zur Herstellung von Immunoassays.

**19.** Verwendung von monoklonalen Antikörpern gemäß den Ansprüchen 1 bis 4 zur Isolierung von atrialen, natriuretischen Peptiden mittels Immunadsorptionschromatographie.

**20.** Monoklonale Antikörper gemäß den Ansprüchen 1 bis 4 zur Verwendung für tierexperimentelle Arbeiten.

**21.** Monoklonale Antikörper gemäß den Ansprüchen 1 bis 4 zur Verwendung für physiologische oder pharmakologische Untersuchungen.

**Claims**

**1.** Monoclonal antibodies against atrial, natriuretic peptides of mammals, characterised in that they have no cross-reactivity with other endogenous mediators having vasoactivity or with effects on the salt-water balance.

2. Monoclonal antibodies according to Claim 1, the endogenous mediators being angiotensin II (human), leu-enkephalin, substance p, aldosterone, ACTH (pig), bradykinin, $\gamma$-MSH, insulin (rat), vasopressin and renin (pig).

3. Monoclonal antibodies according to Claims 1 and 2 produced by the hybridoma cell 85031401.

4. Monoclonal antibodies according to Claims 1 and 2 produced by the hybridoma cell 85031402.

5. Hybridoma cell lines, characterised in that they produce monoclonal antibodies according to Claims 1 to 4.

6. Process for the preparation of monoclonal antibodies according to Claims 1 to 4, characterised in that hybridoma cell lines according to Claim 5 are used.

7. Process according to Claim 6, characterised in that the hybridoma cells are cultivated in a suitable culture medium, and the antibodies are obtained from the supernatant.

8. Process according to Claim 6, characterised in that the hybridoma cells are injected into syngeneic or semisyngeneic mice, and the antibodies are obtained from the blood or the peritoneal exudate.

9. Process for the preparation of hybridoma cells according to Claim 5, characterised in that myeloma cells are fused with cells from mammals which produce antibodies against atrial, natriuretic peptides.

10. Process according to Claim 9, characterised in that spleen cells from mammals which have been immunised against atrial natriuretic peptides are fused with myeloma cells.

11. Process according to Claims 9 and 10, characterised in that spleen cells from mice which have been immunised against atrial natriuretic peptides are fused with myeloma cells.

12. Process according to Claims 9 to 11, characterised in that mouse myeloma cells are used.

13. Process according to Claims 9 to 12, characterised in that the myeloma cells are of the non-secreting type.

14. Process according to Claims 9 to 13, characterised in that the immunisation is carried out with atrial natriuretic peptides which are bound to an immunogenic carrier.

15. Process according to Claims 9 to 14, characterised in that the immunogenic carrier is a protein.

16. Use of monoclonal antibodies according to Claims 1 to 4 for the determination of atrial, natriuretic peptides in biological fluids.

17. Use of monoclonal antibodies according to Claims 1 to 4 for the determination of atrial, natriuretic peptides in blood, plasma, serum, urine, lymph or in the cerebrospinal fluid.

18. Use of monoclonal antibodies according to Claims 1 to 4 for the production of immunoassays.

19. Use of monoclonal antibodies according to Claims 1 to 4 for the isolation of atrial, natriuretic peptides by means of immunoadsorption chromatography.

20. Monoclonal antibodies according to Claims 1 to 4 for use in animal experimental studies.

21. Monoclonal antibodies according to Claims 1 to 4 for use in physiological or pharmacological investigations.

**Revendications**

1. Anticorps monoclonaux dirigés contre des peptides natriurétiques auriculaires de mammifères, caractérisés en ce qu'ils n'ont pas de réactivité croisée avec d'autres médiateurs endogènes agissant sur les vaisseaux ou agissant sur le bilan hydrominéral.

2. Anticorps monoclonaux suivant la revendication 1, les médiateurs endogènes étant l'angiotensine II (humaine), la Leu-encéphaline, la substance P, l'aldostérone, l'ACTH (porc), la bradykinine, la $\gamma$-MSH, l'insuline (rat), la vasopressine et la rénine (porc).

3. Anticorps monoclonaux suivant les revendications 1 et 2, produits par la cellule d'hybridome 85031401.

4. Anticorps monoclonaux suivant les revendications 1 et 2, produits par la cellule d'hybridome 85031402.

5. Lignées cellulaires d'hybridome, caractérisées en ce qu'elles produisent des anticorps monoclonaux suivant les revendications 1 à 4.

6. Procédé de production d'anticorps monoclonaux suivant les revendications 1 à 4, caractérisé en ce qu'on utilise des lignées cellulaires d'hybridome suivant la revendication 5.

7. Procédé suivant la revendication 6, caractérisé en ce qu'on cultive les cellules d'hydridome dans un milieu de culture approprié et on tire les anticorps de la liqueur surnageante.

8. Procédé suivant la revendication 6, caractérisé en ce que les cellules d'hybridome sont injectées à des souris congénitales ou semi-congénitales et on tire les anticorps du sang ou de l'exsudat péritonéal.

9. Procédé de production de cellules d'hybridome suivant la revendication 5, caractérisé en ce qu'on fusionne des cellules de myélome avec des cellules provenant de mammifères qui produisent des anticorps contre des peptides natriurétiques auriculaires.

10. Procédé suivant la revendication 9, caractérisé en ce qu'on fusionne avec des cellules de myélome des cellules spléniques de mammifères immunisés contre des peptides natriurétiques auriculaires.

11. Procédé suivant les revendications 9 et 10, caractérisé en ce qu'on fusionne avec des cellules de myélome des cellules spléniques de souris immunisées contre des peptides natriurétiques auriculaires.

12. Procédé suivant les revendications 9 à 11, caractérisé en ce qu'on utilise des cellules de myélome de souris.

13. Procédé suivant les revendications 9 à 12, caractérisé en ce que les cellules de myélome sont du type non sécréteur.

14. Procédé suivant les revendications 9 à 13, caractérisé en ce que l'immunisation est effectuée avec des peptides natriurétiques auriculaires liés à un support immunogène.

15. Procédé suivant les revendications 9 à 14, caractérisé en ce que le support immunogène est une protéine.

16. Utilisation d'anticorps monoclonaux suivant les revendications 1 à 4 pour le dosage de peptides natriurétiques auriculaires dans des liquides biologiques.

17. Utilisation d'anticorps monoclonaux suivant les revendications 1 à 4 pour le dosage de peptides natriurétiques auriculaires dans le sang, le plasma, le sérum, l'urine, la lymphe ou le liquide céphalorachidien.

18. Utilisation d'anticorps monoclonaux suivant les revendications 1 à 4 pour la réalisation d'immunoessais.

**19.** Utilisation d'anticorps monoclonaux suivant les revendications 1 à 4 pour l'isolement de peptides natriurétiques auriculaires par chromatographie par immunoadsorption.

**20.** Anticorps monoclonaux suivant les revendications 1 à 4, destinés à être utilisés pour des travaux expérimentaux sur des animaux.

**21.** Anticorps monoclonaux suivant les revendications 1 à 4, destinés à être utilisés pour des études physiologiques ou pharmacologiques.

FIG. 1

FIG. 2

FIG. 3a

FIG. 3b

U$_{Na}$· (μmol/min)

FIG. 4a

U$_{Na}$· (μmol/min)

FIG. 4b